# EUROPÄISCHE PATENTSCHRIFT

(11) **EP 0 517 654 B1**
(45) Veröffentlichungstag und Bekanntmachung des Hinweises auf die Patenterteilung: **17.07.1996**
(21) Anmeldenummer: 92810386.0
(22) Anmeldetag: 22.05.1992
(51) Int. Cl.: A61M 29/02

(54) **Kathetersystem zum mechanischen Dilatieren von Koronarstenosen**
Catheter system to mechanically dilate the coronary stenosis
Système de cathétérisme pour dilater mécaniquement les sténoses coronaires

(30) Priorität: 03.06.1991 CH 1635/91
(43) Veröffentlichungstag der Anmeldung: 09.12.1992
(73) Patentinhaber: SCHNEIDER (EUROPE) AG, CH-8180 Bülach (CH)
(72) Erfinder: Niederhauser, Werner, CH-8046 Zürich (CH); Hofmann, Eugen, CH-8064 Zürich (CH); Pfenninger-Ganz, Susanne, CH-8610 Uster (CH)
(74) Vertreter: Groner, Manfred

(56) Entgegenhaltungen:
- EP-A- 0 380 873
- EP-A- 0 394 969
- EP-A- 0 441 384
- US-A- 4 892 519

## Beschreibung

Die Erfindung betrifft ein Kathetersystem nach dem Oberbegriff des Anspruchs 1.

Die perkutane Dilatation von Koronarstenosen mit einem Ballondilatations-Katheter wurde 1976 von A. Grüntzig eingeführt und hat sich als sehr effektives und schonendes Verfahren bewährt. Um die Dilationszeit ohne erhöhte Gefahr einer Ischämie verlängern zu können und damit die Gefahr einer Restenosierung zu mindern, weist der gattungsbildende Ballondilatationskatheter nach der US-A-4,892,519 für die Perfusion von Gefässflüssigkeit vor und nach dem Ballon miteinander verbundene Seitenlöcher auf. Damit bleibt auch während einer Ballondilation ein Blutfluss entsprechend dem natürlichen Druckgefälle enthalten. Dieser Katheter ist zweilumig ausgebildet, wobei ein Lumen den Führungsdraht aufnimmt, der an seinem distalen Ende fest mit dem Katheter verbunden ist. Der fest montierte Führungsdraht verengt die Verbindung zwischen den Seitenlöchern, so dass nicht immer ein hinreichender Fluss der Gefässflüssigkeit gewährleistet ist. Ein einfaches und schnelles Wechseln dieses Katheters ist nicht möglich. Der Erfindung liegt die Aufgabe zugrunde, ein Kathetersystem zu schaffen, das die genannte Schwierigkeit vermeidet und zugleich die Verwendbarkeit erweitert. Diese Aufgabe ist durch die Erfindung gemäss Anspruch 1 gelöst.

Wesentlich ist, dass die erfindungsgemässe Katheteranordnung ähnlich wie diejenige nach der EP-B-0 203 945 (Monorail-System) als auch wie üblich als "über den Draht Katheter" verwendbar ist. Dies erlaubt die folgende vorteilhafte Behandlungsmethode. Die Stenose wird zuerst mit einer Katheteranordnung nach dem Monorail-System behandelt. Nach dieser ersten Behandlung wird der Ballondilatationskatheter entfernt, wobei der Führungsdraht an seiner Stelle verbleibt. Ueber diesen liegenden Führungsdraht wird nun der Ballondilatationskatheter der erfindungsgemässen Anordnung eingeführt, wobei der Führungsdraht wie beim "Monorail-System" vor und nach dem Ballon durch die seitliche Austrittsöffnung und das Endloch in den Katheter ein- bzw. austritt. Hierbei kann nötigenfalls ein Versteifungsdraht in das zweite Lumen des Ballondilatationskatheters eingeführt werden. Damit bei der Dilatation der Stenose ein hinreichender Fluss der Gefässflüssigkeit durch das Ballonsegment hindurch gewährleistet ist, wird der Führungsdraht bis vor die proximal letzte Seitenöffnung zurückgezogen. Diese Position kann mittels einer röntgenographisch sichtbaren Goldmarkierung gefunden werden. Wird nun trotz der Markierung versehentlich der steuerbare Führungsdraht vollständig aus der Austrittsöffnung zurückgezogen, so kann für das Wiedereinführen des Führungsdrahtes die seitliche Austrittsöffnung des liegenden Ballondilatationskatheters nicht mehr gefunden werden. In diesem Fall wird ein Führungsdraht durch das sich über die ganze Länge des Ballondilatationskatheters erstreckende zweite Lumen (Drahtlumen) eingeführt. Der Ballondilationskatheter ist nun also wie üblich und nicht nach dem Monorail-System geführt.

Ein wesentlicher Vorteil des erfindungsgemässen Kathetersystms besteht darin, dass der Führungsdraht ähnlich wie bei dem aus der EP-B-0 203 945 der Anmelderin bekannten "Monorail-Katheter" weitgehend frei von Reibungen nach dem Gleitschienenprinzip bewegbar ist.

Nach einer Weiterbildung der Erfindung weist der Schaft des Ballondilatationskatheters im Bereich der proximal des Ballons angeordneten Seitenlöchern einen grösseren Aussendurchmesser auf als der anschliesende Schaftbereich und ist die Austrittsöffnung für den Führungsdraht in einem stufenförmigen Uebergangsbereich angeordnet. Damit kann die Reibung des Führungsdrahtes am Katheterschaft besonders klein gehalten werden.

Nach einer weiteren vorteilhaften Ausführung ist der Schaft im Querschnitt koaxial. Bei einem solchen besonders steifen Schaft kann ein temporärer Versteifungsdraht entbehrlich sein.

Ausführungsbeispiele der Erfindung werden anhand der Zeichnung näher erläutert. Es zeigen:
- Fig. 1: eine Ansicht eines Ballondilatationskatheters,
- Fig. 2: ein querschnitt durch einen distalen Bereich eines erfindungsgemässen Kathetersystems,
- Fig. 3: eine Ansicht eines proximalen Bereich des erfindungsgemässen Kathetersystems,
- Fig. 4: ein querschnitt entlang der Linie IV-IV in Fig. 2,
- Fig. 5: ein querschnitt entlang der Linie V-V in Fig. 2,
- Fig. 6: ein querschnitt entlang der Linie VI-VI in Fig. 2
- Fig. 7: ein querschnitt entlang der Linie VII-VII in Fig. 2,
- Fig. 8: eine Darstellung eines erfindungsgemässen Kathetersystems gemäss Fig. 2, wobei jedoch der steuerbare Führungsdraht zum Zweck einer ungehinderten Perfusion teilweise zurückgezogen ist,
- Fig. 9: im Längsschnitt den distalen Bereich eines erfindungsgemässen Kathetersystems nach einer Variante,
- Fig. 10: ein Schnitt durch die Verbindungsstelle "Schaft-Ballon" des Kathetersystems nach der Variante,
- Fig. 11: ein Schnitt entlang der Linie XI-XI in Fig. 10, und
- Fig. 12: ein Schnitt entlang der Linie XII-XII in Fig. 10.

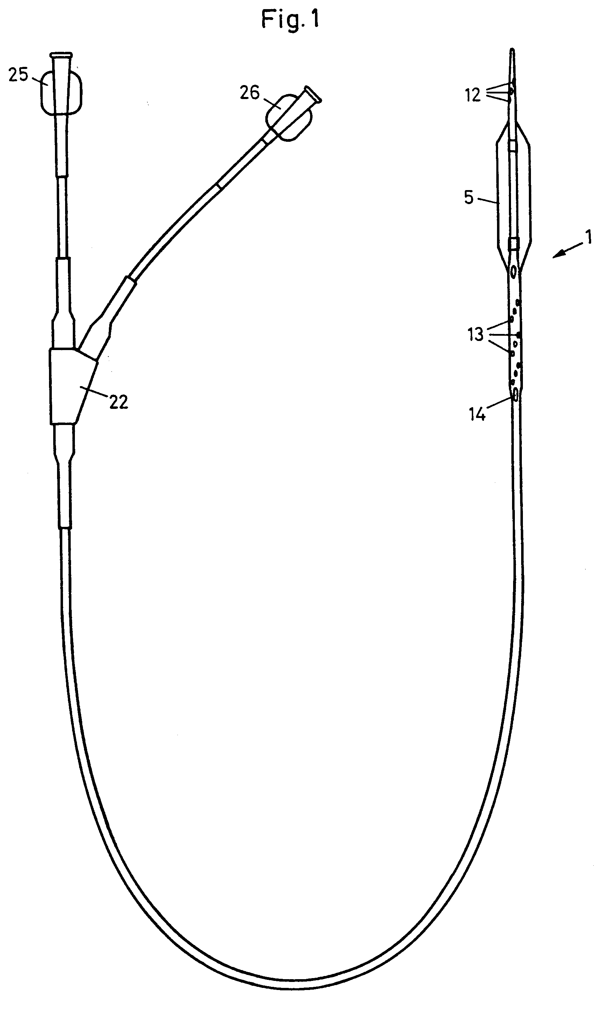

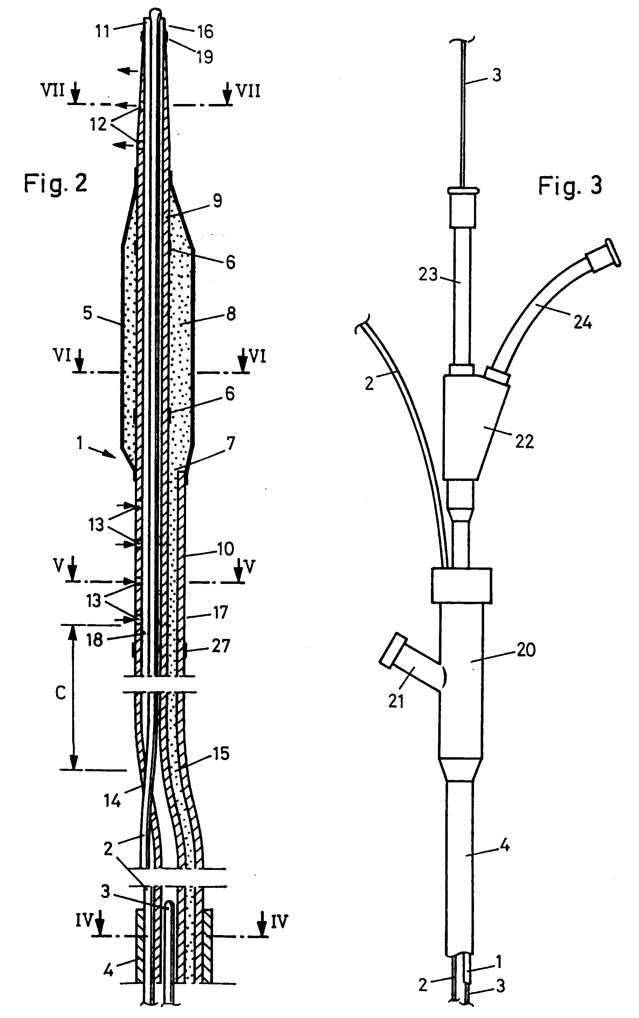

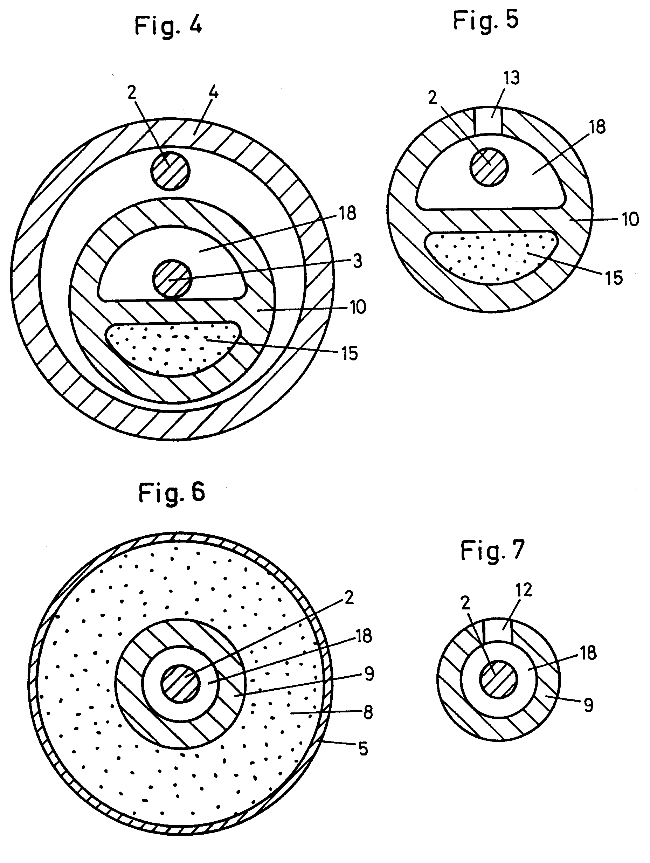

Das erfindungsgemässe Kathetersystem nach den Fig. 1 bis 7 weist einen Ballondilatationskatheter 1, einen Führungskatheter 4, einen temporären Versteifungsdraht 3 sowie einen Führungsdraht 2 auf. Wie die Figuren 2 und 3 zeigen, sind der Versteifungsdraht 3, der Ballondilatationskatheter 1 und der Führungskatheter 4 koaxial zueinander angeordnet. In einem Anschlussstück 20 des Führungskatheters 4 ist eine hier nicht gezeigte Dichtung angeordnet, welche den Führungskatheter 4 gegenüber dem Dilatationskatheter 1 abdichtet. Der Führungsdraht 2 verläuft neben dem Ballondilatationskatheter 1 parallel zu diesem im Führungskatheter 4. Der Versteifungsdraht 3 ist in einer Abzweigung 23 eines Abzweigungsstücks 22 des Dilatationsballonkatheter 1 verschiebbar abgedichtet und endet an seinem distalen Ende vor einer seitlichen Austrittsöffnung 14 des Ballondilatationskatheters 1. Eine weitere Abzweigung 24 des Abzweigungstücks 22 dient zum Dilatieren eines Ballons 5 des Katheters 1 und ist dazu über ein Drucklumen 15 mit diesem verbunden. Für die Injektion von Kontrastmitteln oder Spüllösung ist ferner am Anschlussstück 20 ein seitlicher Anschluss 21 angebracht.

Der Ballon 5 ist in bekannter Weise mit einem zwei-lumigen Schaft 17 des Ballondilatationskatheters 1 verbunden. Wie die Fig. 2 zeigt, treten der Schaft 17 und der Führungsdraht 2 am distalen Ende des Einführungskatheters 4 aus. Distal und proximal des Ballons 5 weist der Schaft 17 jeweils mehrere Seitenöffnungen 12 bzw. 13 auf. Diese Oeffnungen 12 und 13 sind über ein Lumen 18 des Schaftes 17 miteinander verbunden. Diese Seitenöffnungen erlauben auch während einer Dilatation bei entsprechendem Druckunterschied vor und nach der Stenose eine Perfusion von Blutflüssigkeit.

Im Bereich der proximalen Seitenöffnungen 13 ist der Aussendurchmesser des Schaftes 10 in einem Bereich C grösser als vor der Austrittsöffnung 14. Die Austrittsöffnung 14 ist wie aus Fig. 2 ersichtlich an einem stufenförmigen Absatz des Schaftes 17 angeordnet. Die Austrittsöffnung 14 hat wie aus Fig. 1 ersichtlich die Form eines Längsschlitzes.

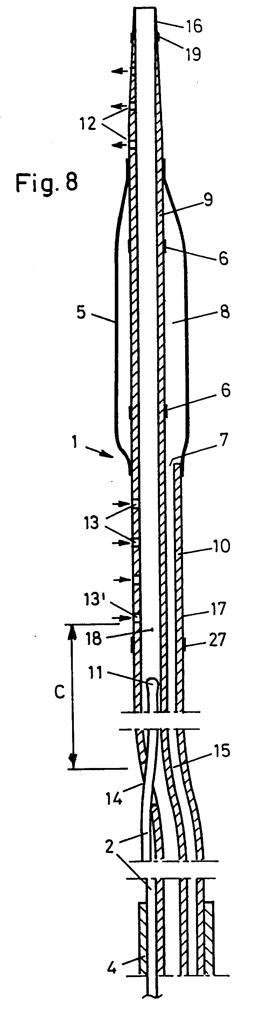

Damit während einer Dilatation einer Stenose für Gefässflüssigkeit eine ungehinderte Perfusion möglich ist, wird der steuerbare Führungsdraht 2, wie in Fig. 8 gezeigt, soweit zurückgezogen, dass sein distales Ende 11 proximal der Seitenöffnung 13′ liegt, die am weitesten von einem Endloch 16 des Dilatationskatheters entfernt ist. Mittels einer Goldmarkierung 27 kann die geeignete Position für das Ende 11 des Führungsdrahtes 2 röntgenographisch sichtbar gemacht werden. Der Abstand C zwischen der Seitenöffnung 13' und der Austrittsöffnung 14 ist vergleichsweise gross gewählt, und beträgt beispielsweise 25-30 cm. Damit ist gewährleistet, dass der im distalen Bereich sehr schmale und äusserst flexible Führungsdraht 2 nach seinem teilweisen Rückzug wieder in den Ballon eingeschoben werden kann.

Zur Lokalisierung des Dilatationsballons sind am Schaft 9 zwei röntgendichte Markierungsringe 6 angebracht.

Der temporäre Versteifungsdraht 3 ist ein einfacher Draht, der auf der Aussenseite zur Verminderung der Gleitreibung teflonisiert sein kann. Seine Länge beträgt beispielsweise 1,20 m. Der Aussendurchmesser des Schaftes 17 beträgt im Bereich der Seitenlöcher 13 vorzugsweise etwa 1,5 mm und proximal vor der Austrittsöffnung 14 etwa 1,4 mm. Der Schaft 17 ist ohne Versteifungsdraht 3 vergleichsweise weich.

Zum Dilatieren einer Stenose wird der Führungskatheter 4 perkutan über beispielsweise eine Beinarterie zur Stenose vorgeführt. Durch den Führungskatheter 4 wird nun der Führungsdraht 2 vorgeführt, bis sein distales Ende distal der Stenose liegt. Wesentlich ist, dass aufgrund der geringen Reibung des Führungsdrahtes dieser frei und kaum behindert steuerbar ist. Liegt der Führungsdraht 2 mit seinem distalen Ende in der Stenose, so wird der Ballondilatationskatheter 1 auf den Führungsdraht 2 aufgeschoben und auf diesen gleitend soweit vorgeschoben, bis der Ballon 5 in der Stenose liegt. In bekannter Weise wird nun der Ballon 5 mittels der Druckflüssigkeit 8 dilatiert. Beim Verschieben des Ballondilatationskatheters und auch im Fall eines Katheterwechsels ist der Führungsdraht 2 an seinem proximalen Ende fixiert. Da wie oben erwähnt durch die Seitenlöcher 12 und 13 auch während eine Dilatation eine Perfusion der Blutflüssigkeit erfolgt, ist mit dem erfindungsgemässen Kathetersystem eine Dilatation über einen längeren Zeitraum möglich. Sollte die Perfusion durch an den Löchern 12 und 13 geronnenes Blut verhindert sein, so können diese Oeffnungen über das Lumen 18 beispielsweise mit einer Heparinlösung gespült werden.

Damit während der Dilatation die Perfusion durch den Führungsdraht 2 nicht gehindert ist, wird der Führungsdraht 2 ganz oder teilweise aus dem Ballonsegment zurückgezogen. Hat der Führungsdraht die seitliche Austrittsöffnung verlassen, so kann er nicht mehr in das Ballonsegment eingeführt werden. Der Führungsdraht 2 wird deshalb durch das Abzweigungsstück 23 bzw. 25 in das zweite Lumen 18 eingeführt.

Bei einem bevorzugten Behandlungsverfahren wird die Stenose in einem ersten Schritt mit einem Katheter nach dem "Monorail-System" dilatiert. Nach dem entfernen des "Monorail"-Ballondilatationskatheters wird auf den bereits liegenden Führungsdraht 2 der Ballondilatationskatheter 1 aufgeschoben, wobei der Führungsdraht den Katheter 1 durch die Austrittsöffnung 14 verlässt, der Katheter 1 somit nach dem Gleitschienenprinzip verschiebbar ist. Der Katheter 1 kann für seine Einführung mit dem temporären Versteifungsdraht 3 versehen sein. Vor dem dilatieren der Stenose mit dem Katheter 1 wird der Führungsdraht 2 zur Verbesserung der Perfusion zurückgezogen. Ein neuer Führungsdraht 2 wird durch das Abzweigungsstück 23 bzw. 25 eingeführt, sobald ein solcher für das weitere Verfahren notwendig sein sollte.

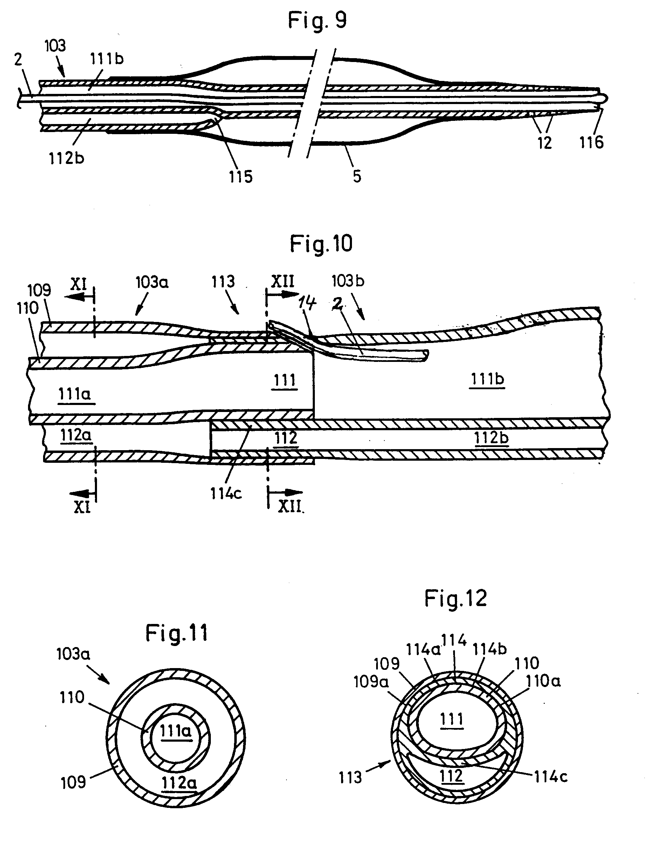

Die Figuren 9 bis 12 zeigen eine Ausführung des erfindungsgemässen Kakthetersystems, das sich von der oben erwähnten insbesondere dadurch unterscheidet, dass ein im querschnitt koaxialer und vergleichsweise steifer Schaft 103 vorgesehen ist. Dies hat den Vorteil, dass ein temporärer Versteifungsdraht entbehrlich sein kann.

Der Führungsdraht 2 ist in einem durchgehenden ersten Lumen 111 des Dilatationskatheters in Längsrichtung verschiebbar. Der Innenraum des Ballons 5 ist über ein zweites Lumen 112 mit einer hier nicht gezeigten Druck-Saugeinrichtung verbunden.

Der flexible Schaft 103 ist an seinem distalen Ende mit dem Ballon 5 verbunden. Die Fig. 9 zeigt den Ballon 5, der über eine Oeffnung 115 mit dem zweiten Lumen 112 verbunden ist. Mittels der genannten Druck-Saugeinrichtung kann der Ballon 8 zu seiner Einführung in ein Gefäss gefaltet und zur Behandlung einer Stenose dilatiert werden. Der Führungsdraht 13 kann, wie in Fig. 9 gezeigt, an seinem distalen Ende durch eine Oeffnung 116 über den Dilatationsballon 5 hinaus vorgeschoben werden. Er ist wie bekannt an seinem distalen Ende besonders flexibel und dennoch torsionssteif.

Der Schaft 103 ist aus einem proximalen Abschnitt 103a und einem distalen Abschnitt 103b hergestellt. Diese Abschnitte 103a und 103b sind separat hergestellt und an der Verbindungsstelle 113 miteinander verbunden. Wie die Figuren 11 und 12 zeigen, sind die querschnitte der beiden Abschnitte 103a und 103b verschieden. Der proximale Abschnitt 103a besteht aus zwei koaxialen Schlauchstücken 109 und 110, wobei das innere Schlauchstück 110 ein Lumen 111a für den Führungsdraht 13 bildet. Der Zwischenraum zwischen den Schlauchstücken 109 und 110 bildet ein Lumen 112a, in welchem Druckflüssigkeit zwischen dem Ballon 5 und der Druck-Saugpumpe zirkulieren kann. Die Schlauchstücke 109 und 110 besitzen einen kreisrunden querschnitt und bestehen aus einem thermoplastischen Kunststoff.

Der Schaftabschnitt 103b besteht aus einem zweilumigen, extrudierten Schlauchstück, das in bekannter Weise mit dem Ballon 5 verbunden ist. Ein erstes annähernd kreisförmiges Lumen 111b dient zur Aufnahme des Führungsdrahtes 13 und ein zweites halbmondförmiges Lumen 112b dient zur Aufnahme der Druckflüssigkeit. Beide Lumen sind durch eine am Abschnitt 103b angeformte Trennwand 114c voneinander getrennt.

Die Abschnitte 103a und 103b sind gemäss Fig. 10 derart miteinander verbunden, dass die Lumen 111a und 111b das erste Lumen 111 und die Lumen 112a und 112b das Lumen 112 bilden. Die Lumen 111 und 112 sind somit auch an der Verbindungsstelle 113 vollständig voneinander getrennt. Um die separat hergestellten Abschnitt 103a und 103b miteinander zu verbinden, werden diese zusammengeschoben, bis sich die entsprechenden Enden auf einer Länge von wenigen Millimetern überlappen.

Im Bereich der Verbindungsstelle 113 sind die beiden Schlauchabschnitte 103a und 103b vorzugsweise miteinander verschweisst. Vorzugsweise ist die Aussenfläche 114a des Schlauchstücks 114 mit der Innenfläche 109a des Schlauchstücks 109 und die Aussenseite 110a des Schlauchstückes 110 mit der Innenseite 114b des Schlauchstückes 114 verschweisst. Möglich ist auch eine Ausführung, bei welcher das Schlauchstück 114 an dem zu verbindenden Ende eine verminderte Wandstärke aufweist. Diese verminderte Wandstärke kann hier beispielsweise auch durch Abschleifen des Schlauchstückes 114 an der Aussenseite 114a erfolgen. Denkbar ist auch eine Ausführung, bei welcher die beiden Abschnitt 103a und 103b am überlappenden Bereich 113 miteinander verklebt sind.

Sind die Abschnitte 103a und 103b aus gleichem oder ähnlichem Kunststoff hergestellt und sind die Wandstärken annähernd gleich, so besitzt das Schlauchstück 103a eine grössere Steifigkeit als das Schlauchstück 103b. Der Hauptgrund der unterschiedlichen Steifigkeit wird darin gesehen, dass das Schlauchstück 103a im Längsschnitt gemäss Fig. 10 aus vier Wandbereichen und der Abschnitt 103b lediglich aus drei Wandbereichen besteht. Die unterschiedliche Steifigkeit ist somit in erster Linie durch die unterschiedlichen Strukturen der beiden Abschnitte 103a und 103b verursacht.

Die Länge des flexibleren Abschnittes 103b ist vorzugsweise an die Länge des bogenförmigen Abschnittes des zu behandelnden Blutgefässes angepasst.

In dem steifen aber weitgehend gerade verlaufenden Abschnitt 103a gleitet der Führungsdraht 13 besonders leicht, da das Lumen 111a weitgehend kreisrund ist. Dies kann durch geeignete Beschichtungen des Führungsdrahtes 13 und der Innenseite des Schlauchstückes 110 noch verbessert werden.

Die Ausführung nach den Figuren 9 bis 12 hat den Vorteil, dass er vergleichsweise hart und damit beim Einführen weniger knickanfällig ist. Der Schaft ist zudem dünnwandiger, weshalb der Ballon schneller gefüllt und entleert werden kann, was bei einer operativen Behandlung selbstverständlich sehr wesentlich ist.

Die Austrittsöffnung 14 für den Führungsdraht 2 ist vorzugsweise im Ueberlappungsbereich der beiden Schaftabschnitte 103a und 103b angordnet, wie in Fig. 10 gezeigt. Durch die distal des Ueberlappungsbereichs anschliessende Schalter des Schaftes wird ein vergleichsweise flacher Verlauf des Führungsdrahtes 2 erreicht.

Das erfindungsgemässe Kathetersystem eignet sich besonders als sogenannter "Notfallkatheter", somit dann, wenn nach einer Dissektion eine vergleichweise langdauernde Dilatation notwendig ist.

## Patentansprüche

1. Kathethersystem zum mechanischen Dilatieren insbesondere von Koronarstenosen, mit einem Ballondilatationskatheter (1) und einem steuerbaren Führungsdraht (2), wobei der Ballondilatationskatheter (1) für die Perfusion von Gefässflüssigkeit während einer Dilation distal und proximal des Ballons (5) Seitenöffnungen (12, 13) aufweist, die über ein im Ballon (5) sich erstreckendes Lumen (18) miteinander verbunden sind, wobei ein Schaft (17) des Ballondilationskatheters (1) zwei-lumig ausgebildet ist und ein erstes Lumen (15) für eine Druckflüssigkeit (8) zum Aufweiten des Ballons (5) und das zweite Lumen (18) zur Aufnahme des Führungsdrahtes (2) und zum Spülen der Seitenöffnungen (12, 13) vorgesehen sind, dadurch gekennzeichnet, dass proximal des Ballons (5) eine Austrittsöffnung (14) und distal ein Endloch (16) für den Führungsdraht (2) vorgesehen sind und dass ein Führungskatheter (4) vorgesehen ist.

2. Kathetersystem nach Anspruch 1, dadurch gekennzeichnet, dass der Schaft (17) des Ballondilatationskatheters (1) im Bereich der proximal des Ballons (5) angeordneten Seitenöffnungen (13) einen grösseren Aussendurchmesser aufweist als ein proximal anschliessender Schaftbereich und dass die Austrittsöffnung (14) für den Führungsdraht (2) in einem stufenförmigen Uebergangsbereich angeordnet ist.

3. Kathetersystem nach Anspruch 1 oder 2, dadurch gekennzeichnet, dass die Einführungsöffnung (14) die Form eines Längsschlitzes aufweist.

4. Kathetersystem nach einem der Ansprüche 1 bis 3, dadurch gekennzeichnet, dass der Schaft (17) des Ballondilatationskatheters (19) im Bereich der Austrittsöffnung (14) farblich markiert ist.

5. Kathetersystem nach einem der Ansprüche 1 bis 4, dadurch gekennzeichnet, dass der Abstand (C) zwischen der Austrittsöffnung (14) und der nächstliegenden Seitenöffnung (13') so gewählt ist, dass bei einem teilweisen Rückzug des Führungsdrahtes (2) dieser wieder zurückgeschoben werden kann.

6. Kathetersystem nach Anspruch 5, dadurch gekennzeichnet, dass der Abstand (C) 20-30 cm beträgt.

7. Kathetersystem nach einem der Ansprüche 1 bis 6, dadurch gekennzeichnet, dass der Ballondilatationskatheter (1) zur Lokalisierung der geeigneten Position der Spitze (11) des Führungsdrahtes (2) bei einem teilweisen Rückzug desselben eine röntgendichte Markierung (27) aufweist.

8. Kathetersystem nach einem der Ansprüche 1 bis 7, dadurch gekennzeichnet, dass der Schaft (103) des Ballondilatationskatheters aus wenigstens zwei separat hergestellten und miteinander verbundenen Abschnitten (103a,103b) besteht, wobei ein proximaler Abschnitt (103a) aus zwei koaxialen Schlauchstücken (109,110) und ein flexiblerer distaler Abschnitt (103b) aus einem zweilumigen Schlauchstück (114) hergestellt sind und die Abschnitte (103a,103b) derart miteinander verbunden sind, dass das erste Lumen (111b) des distalen Abschnitts (103b) mit dem Lumen (111a) des koaxialen inneren Schlauchstücks (110) des proximalen Abschnitts (103a) und das zweite Lumen (112b) des distalen Abschnitts (103b) mit dem Lumen (112a) zwischen den beiden koaxialen Schlauchstücken (109,110) des proximalen Abschnitts (103a) verbunden ist.

9. Kathetersystem nach Anspruch 8, dadurch gekennzeichnet, dass die beiden Abschnitte (103a,103b) sich an ihrer Verbindungstelle (113) überlappen und an den anliegenden Flächen miteinander verschweisst sind.

10. Kathetersystem nach Anspruch 8 oder 9, dadurch gekennzeichnet, dass wenigstens ein Abschnitt (103a) an dem mit dem anderen Abschnitt (103b) verbundenen Ende eine verminderte Wandstärke aufweist.

## Claims

1. Catheter system for the mechanical dilation, in particular of coronary stenoses, with a balloon dilation catheter (1) and a controllable guide wire (2), wherein the balloon dilation catheter (1) has, for the perfusion of vessel fluid during dilation, lateral orifices (12, 13) which are arranged distally from and proximally to the balloon (5) and are connected to one another via a lumen (18) extending in the balloon (5), a shank (17) of the balloon dilation catheter (1) being designed with two lumens, a first lumen (15) being provided for a pressure fluid (8) for expansion of the balloon (5) and the second lumen (18) being provided to receive the guide wire (2) and to flush the lateral orifices (12, 13), characterized in that an outlet orifice (14) is provided proximally to the balloon (5) and an end hole (16) for the guide wire (2) distally therefrom and in that a guide catheter (4) is provided.

2. Catheter system according to Claim 1, characterized in that the shank (17) of the balloon dilation catheter (1) has a greater external diameter in the region of the lateral orifices (13) arranged proximally to the balloon (5) than a proximally adjoining shank region and in that the outlet orifice (14) for the guide wire (2) is arranged in a step-shaped transitional region.

3. Catheter system according to Claim 1 or 2, characterized in that the introduction orifice (14) has the form of a slot.

4. Catheter system according to one of Claims 1 to 3, characterized in that the shank (17) of the balloon dilation catheter (19) is marked in colour in the region of the outlet orifice (14).

5. Catheter system according to one of Claims 1 to 4, characterized in that the distance (C) between the outlet orifice (14) and the closest lateral orifice (13') is selected such that, during a partial retraction of the guide wire (2), the guide wire can be pushed back again.

6. Catheter system according to Claim 5, characterized in that the distance (C) is 20 to 30 cm.

7. Catheter system according to one of Claims 1 to 6, characterized in that the balloon dilation catheter (1) has an X-ray-resistant marking (27) for locating the appropriate position for the tip (11) of the guide wire (2) during a partial retraction thereof.

8. Catheter system according to one of Claims 1 to 7, characterized in that the shank (103) of the balloon dilation catheter consists of at least two separately produced portions (103a, 103b) which are connected to one another, a proximal portion (103a) being produced from two coaxial pieces of tube (109, 110) and a more flexible distal portion (103b) from a two-lumen piece of tube (114) and the portions (103a, 103b) being connected to one another in such a way that the first lumen (111b) of the distal portion (103b) is connected to the lumen (111a) of the coaxial internal piece of tube (110) of the proximal portion (103a) and the second lumen (112b) of the distal portion (103b) is connected to the lumen (112a) between the two coaxial pieces of tube (109, 110) of the proximal portion (103a).

9. Catheter system according to Claim 8, characterized in that the two portions (103a, 103b) overlap at their connecting point (113) and are welded to one another at the adjoining faces.

10. Catheter system according to Claim 8 or 9, characterized in that at least one portion (103a) has a reduced wall thickness at the end connected to the other portion (103b).

## Revendications

1. Système à cathéter pour la dilatation mécanique notamment de sténoses coronaires, comportant un cathéter à ballonnet dilatable (1) et un fil de guidage commandable (2), le cathéter à ballonnet dilatable (1) possédant, pour la perfusion d'un liquide d'un vaisseau, pendant une dilatation, des ouvertures latérales (12,13) situées sur le côté distal et sur le côté proximal du ballon (5) et qui sont reliés entre elles, par l'intermédiaire d'une lumière (18) qui s'étend dans le ballon (5), une tige (17) du cathéter à ballonnet dilatable (1) étant agencée de manière à comporter deux lumières, une première lumière (15) étant prévue pour un liquide sous pression (8) servant à dilater le ballon (5), et la seconde lumière (18) étant prévue pour recevoir le fil de guidage (2) et pour le balayage des ouvertures latérales (12,13), caractérisé en ce qu'une ouverture de sortie (14) est prévue sur le côté proximal du ballon (5) et qu'un trou terminal (16) est prévu sur le côté distal pour le fil de guidage (2), et qu'il est prévu un cathéter de guidage (4).

2. Système de cathéter selon la revendication 1, caractérisé en ce que la tige (17) du cathéter à ballonnet dilatable (1) possède, dans la zone des ouvertures latérales (13) situées sur le côté proximal du ballonnet (5), un diamètre extérieur plus important que celui d'une partie de la tige se raccordant sur le côté proximal et que l'ouverture de sortie (14) pour le fil de guidage (2) est disposée dans une zone de jonction de forme étagée.

3. Système de cathéter selon la revendication 1 ou 2, caractérisé en ce que l'ouverture d'introduction (14) possède la forme d'une fente longitudinale.

4. Système de cathéter selon l'une des revendications 1 à 3, caractérisé en ce que la tige (17) du cathéter à ballonnet dilatable (19) est marquée par une couleur dans la zone de l'ouverture de sortie (14).

5. Système de cathéter selon l'une des revendications 1 à 4, caractérisé en ce que la distance (C) entre l'ouverture de sortie (14) et l'ouverture latérale la plus proche (13') est choisie de telle sorte que dans le cas d'un recul partiel du fil de guidage (2), ce dernier peut être à nouveau repoussé.

6. Système de cathéter selon la revendication 5, caractérisé en ce que la distance (C) est égale à 20-30 cm.

7. Système de cathéter selon l'une des revendications 1 à 6, caractérisé en ce que le cathéter à ballon dilatable (1) possède un marquage (27) opaque aux rayons X, pour la localisation de la position appropriée de la pointe (11) de la tige de guidage (2) dans le cas d'un recul partiel de ce fil.

8. Système de cathéter selon l'une des revendications 1 à 7, caractérisé en ce que la tige (103) du cathéter à ballonnet dilatable est constituée par au moins deux sections (103a, 103b) qui sont fabriquées séparément et sont reliées entre elles, une section proximale (103a) étant constituée par deux éléments coaxiaux de tuyau (109,110) et une section distale plus flexible (103b) étant constituée par un élément de tuyau (114) possédant deux lumières, et les sections (103a,103b) étant reliées entre elles de telle sorte que la première lumière (111b) de la section distale (103b) est reliée à la lumière (111a) de l'élément de tuyau intérieur coaxial (110) de la section proximale (103a) et que la seconde lumière (112b) de la section distale (103b) est reliée à la lumière (112a) entre les deux éléments de tuyau coaxiaux (109,110) de la section proximale (103a).

9. Système de cathéter selon la revendication 8, caractérisé en ce que les deux sections (103a,103b) se chevauchent au niveau de leur point de jonction (113) et sont soudées entre elles au niveau des surfaces contiguës.

10. Système de cathéter selon la revendication 8 ou 9, caractérisé en ce qu'au moins une section (103a) possède, sur l'extrémité reliée à l'autre section (103b), une épaisseur de paroi réduite.
